# EUROPEAN PATENT SPECIFICATION

(11) **EP 3 534 776 B1**
(45) Date of publication and mention of the grant of the patent: **06.05.2026**
(21) Application number: 17825594.9
(22) Date of filing: 07.11.2017
(51) Int. Cl.: A61B 5/00, A61B 5/1455

(54) **SENSOR HOUSING AND ASSOCIATED DISPOSABLE ELEMENT**
SENSORGEHÄUSE UND ZUGEHÖRIGES EINWEGELEMENT
BOÎTIER DE CAPTEUR ET ÉLÉMENT JETABLE ASSOCIÉ

(30) Priority: 07.11.2016 GB 201618756
(43) Date of publication of application: 11.09.2019
(73) Proprietor: Newcastle-Upon-Tyne Hospitals NHS Trust, Newcastle-upon-Tyne, Tyne and Wear NE7 7DN (GB)
(72) Inventor: ALLEN, John, Newcastle-upon-Tyne Tyne and Wear NE7 7DN (GB); BEALE, Thomas Anthony William, Newcastle-upon-Tyne Tyne and Wear NE7 7DN (GB); WIGHTMAN, James Patrick, Newcastle-upon-Tyne Tyne and Wear NE7 7DN (GB)
(74) Representative: Elsworth, Dominic Stephen
(86) International application number: PCT/GB2017/053342
(87) International publication number: WO 2018/083503

(56) References cited:
- US-A- 4 274 418
- US-A- 4 303 076
- US-A1- 2012 330 126
- US-A1- 2014 303 462
- US-B1- 6 345 191

## Description

### Field of the Invention

The present invention relates to a medical sensor housing and in particular to an optical sensor housing for use in reflection mode photoplethysmography.

### Background of the Invention

The technique of contact photoplethysmography (PPG) is used *inter alia* in the assessment of peripheral arterial disease. The technique requires optical sensor housings to be attached to the skin of a patient. Light emitted by a near infra-red emitter or an emitter emitting visible wavelength green or red light is scattered and absorbed by the microvasculature of the patient's body at the position of attachment of the sensor housing and back-scatter detected by an adjacent detector or set of detectors located within the sensor housing, the detector configured to detect the wavelength of light emitted by the emitter. PPG sensors are classified as either transmission mode or reflection (also known as adjacent) mode. It is known that reflection mode PPG can offer much more flexibility in where the sensor can be placed on the skin surface than do transmission mode sensors.

The sensor housings used in photoplethysmography are typically attached to the extremities of the patient's body, for example to the great toe / finger pad and ear lobe where pulses can usually be sensed easily. The most used sensor housings are in the form of a clip. One such clip is described in WO97/20497. Typically, such clips are intended for reuse.

To optimise the effectiveness of the photoplethysmography technique the applicants have found that applying a controlled pressure on the skin can be beneficial. Where the sensor exerts a controlled pressure on the skin, movement artefacts in the sensor output signal are reduced and probe amplitude increase is possible. US2014/0303462 describes a device for applying a sensor to a measurement site on a patient's skin. US2012/0330126 describes a monitoring device for attachment to a patient's skin.

US6345191 describes a sensor device which includes a sensor film applied to the skin and a CCD camera.

US4274418 describes a sensor housing for attachment to a patient's skin using an adhesive membrane.

US4303076 describes a probe for transcutaneous blood gas analysis.

### Summary of the Invention

According to the disclosure there is provided a sensing device comprising a sensor housing mounting sensor elements and an attachment member, the attachment member comprising first and second components, the first component configured to receive the sensor housing therein and to permit rotational movement between the attachment member and the sensor housing and to restrict relative movement between the attachment member and the sensor housing in the axial direction of the sensor housing, and the second component comprising a flexible membrane that is attachable to an underside of the first component, the flexible membrane extending at least in part beyond an outer edge of the first component, the flexible membrane comprising attachment means for attachment of the attachment member to a patient, and wherein the attachment member is adapted to allow passage of non-ionising radiation from the sensor housing to the patient.

Preferably, the non-ionising radiation is optical radiation or ultrasound radiation.

The attachment means may comprise at least one hook and loop type fastener.

Alternatively, the attachment means may comprise an adhesive surface at least to the side of the flexible membrane that is distal from the first component. The adhesive of the adhesive surface to the side of the flexible membrane that is distal from the first component preferably has an adhesion within a certain range, and adhesion is defined by a peel adhesion strength to steel in the range of 0.1-1.0kg/25mm width when adhered to steel (as measured using 3M measurement procedure "Adhesion to Steel, 180deg peel"). More preferably, the adhesion defined by a peel adhesion strength to steel is within the range 0.125kg/25mm width to 0.6kg/25mm width.

Preferably, the part of the flexible membrane that extends beyond the outer edge of the first component comprises a plurality of tabs and more preferably at least two tabs, and still more preferably four tabs. The tabs may be spaced equidistantly. Where four tabs are provided they may be provided in pairs, each tab of a pair lying on the same axis. One pair of tabs may be more flexible than the other. For example, the tabs that are for wrapping around the digit may be very flexible, whereas the other pair of tabs may be relatively stiff. The tabs may include a series of graduated markings to aid placement of the device on the patient.

The attachment member may comprise a third component in the form of clip means, wherein the third component is shaped and dimensioned to receive the first component in a push-fit arrangement, and wherein, in use, the flexible membrane is located in-between the first component and the third component. Where the attachment member includes a third component, the flexible membrane attaches the third component to the patient and the first component is attachable to the third component in a push-fit arrangement. The third component also effectivly attaches the flexible membrane to the underside of the first component.

Alternatively, the flexible membrane may be attached to the underside of the first component by adhesive.

Preferably, the flexible membrane has an adhesive surface at least in part to the side thereof that is proximal from the first component. Where the flexible membrane includes a plurality of tabs, or otherwise extends beyond the edge of the first component, such tabs or extensions do not have an adhesive surface to the side that is proximal to the first component.

The flexible membrane may comprise two flexible layers, a first layer for attachment to the underside of the first component and a second layer attached to the first layer and for attachment to the skin of a patient. Preferably, the first layer is provided with adhesive to both sides thereof. The adhesion of the surfaces of the first layer may be defined by a peel adhesion to low density polyethylene (LDPE) of 1.1kg/25.4mm to 1.5kg/25.4mm, for example 1.3kg/25.4mm.

Rather than attaching the flexible membrane to the underside of the first component by adhesive, the two parts may be joined using ultrasonic welding or other suitable techniques.

Advantageously, the flexible membrane provides an unbroken surface. Alternatively, the flexible membrane may have a hole centrally therein and positioned and dimensioned to align with the part of the sensor housing that mounts the sensor elements.

The flexible membrane at least in the region where radiation, for example infra-red light, is to pass exhibits low absorption of the wavelength of radiation passing.

The first component configured to receive the sensor housing therein and to permit rotational movement between the attachment member and the sensor housing and to restrict relative movement between the attachment member and the sensor housing in the axial direction of the sensor housing may comprise a plurality of clips which are resilient and which are so shaped and dimensioned as to grip the sensor housing when the sensor housing is mounted within the clips. Preferably, the clips are sufficiently resilient to allow the sensor housing to ride over free ends of the clips when the sensor housing is pressed into or out of the first component of the attachment member.

The first component of the attachment member may include a hole configured to receive a protruding part of the sensor housing.

When the sensor housing is mounted in the first component of the attachment member a protruding part of the sensor housing may extend through the hole in the first component. The protruding part of the sensor housing may extend beyond the surface of the first component that is distal from the sensor housing. More preferably, the flexible membrane is attached to the underside of the first component around the hole and the flexible membrane covers the hole.

The device of the invention provides a secure fixation of a sensor to the skin of a patient and minimises torque applied to the skin as a result of the weight of the sensor housing and components attached thereto. Further, the device can provide for the application of a controlled pressure on the skin of the patient. The device also provides a physical barrier between the device and the skin of the patient, which allows the sensor housing and electronic components mounted therein to be re-used, the whole attachment member or the second component thereof being disposable. The device of the invention is particularly useful in photoplethysmography, including bilateral PPG where symmetrical attachment of the probes to sites can reduce uncertainty in patient measurements. However, other sensors may be used. For example the sensor housing may house an ultrasound device or a laser Doppler flowmetry probe or a tissue oxygen sensor.

### Brief Description of the drawings

In the Drawings, which illustrate preferred embodiments of the medical sensor housing and associated disposable element of the invention, and are by way of example:
Figure 1 is a schematic representation of a device in place on the finger pad of a patient;
Figure 2 is a schematic representation of the disposable element;
Figure 3 is a schematic representation of the optical sensor housing;
Figure 4 is a cross-section through the sensor housing when attached to the disposable element;
Figure 5 is a cross-section through the disposable element;
Figure 6a is a schematic representation from an underside orientation of an alternative form of the disposable element;
Figure 6b is a schematic representation from the side of the disposable element illustrated in Figure 6a;
Figure 6c is a schematic representation from above of the disposable element illustrated in Figures 6a and 6b;
Figure 7 is a schematic representation of the sensor used with the disposable element illustrated in Figures 6a to 6c;
Figure 8 is a schematic representation of a plan view of an alternative form of flexible membrane...
Figure 9a is a schematic representation of an alternative disposable element;
Figure 9b is a schematic representation of a first component part of the disposable element of Figure 9a;
Figure 9c is a schematic representation of a third component part of the disposable element of Figure 9a; and
Figure 9d is a schematic representation of the component part of Figure 9c in conjunction with a second component part of the disposable element of Figure 9a.

### Detailed Description of the Preferred Embodiments

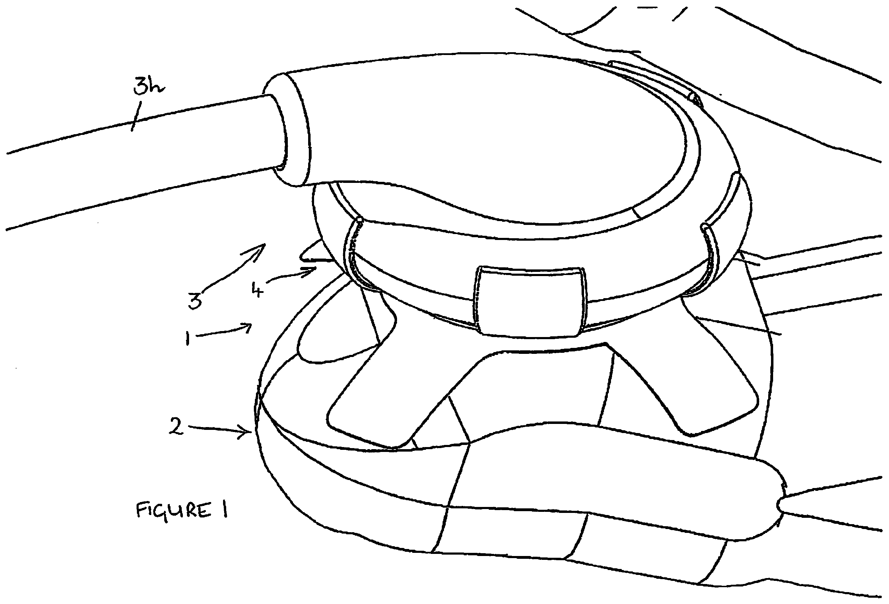

Referring now to Figures 1 to 4, the device 1 is attached to a finger 2 of a patient. The device 1 comprises a sensor housing 3 which is mounted in an attachment member 4. The attachment member 4 comprises two principal components, those being a first component 5 which attaches to the sensor housing 3 and which is formed from a stiff plastic and a second component 6 which is formed from a flexible membrane.

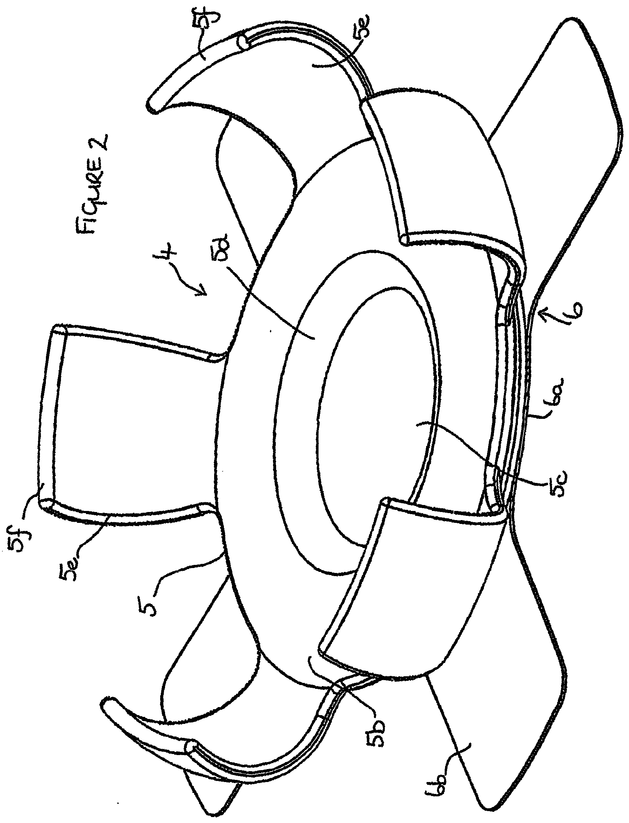

The first component 5 is in the form of a clam shell and has a base 5b which has a central aperture 5c therein. The wall 5d of the aperture 5c is chamfered to match the shape of the part of the sensor housing 3 that mates with the aperture 5c. To the outside of the base 5b is a plurality of clips 5e, five in the illustrated example. The clips are so shaped and dimensioned as to grip the walls of the sensor housing 3. In the illustrated example, the component 5 is a one piece plastic moulding, the clips 5e being sufficiently resilient such that when the sensor housing 3 is pressed against the ends 5f of the clips 5e, the clips 5e bend outwards to permit the walls of the sensor housing 3 to ride over the ends 5f and then return substantially to the position shown in Figure 2 exerting a gripping force on the sensor housing. When it is desired to remove the sensor housing 3 from the first component 5, the sensor housing 3 is pulled out of engagement with the clips 5e, the clips 5e moving outward to facilitate said removal.

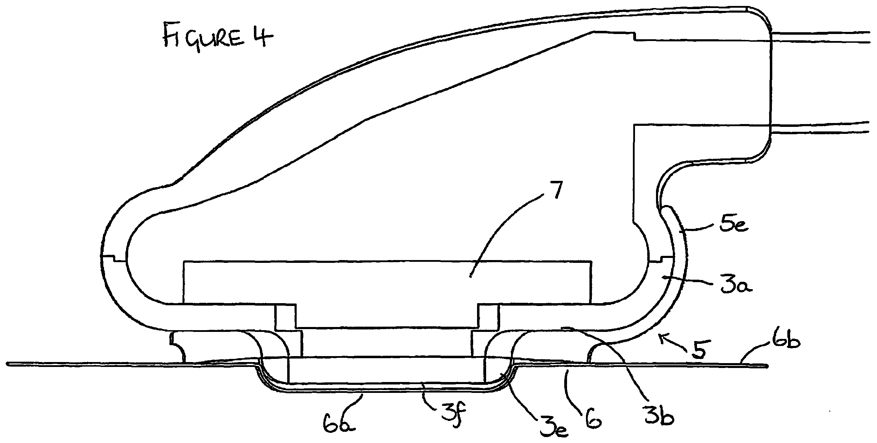

The second component 6 comprises a flexible membrane which is attached to the underside of the first component 5 and which includes a plurality, four in the present example, of tabs 6b extending from a central part 6a, which is described in greater below in relation to Figure 4.

The underside of the second component 6 has an adhesive surface to provide for attachment to the skin of a patient. The adhesive surface may be provided by an adhesive layer applied to the underside of the second component 6, or by forming at least one layer of the second component of a material that is impregnated with adhesive. A peel off layer covering the underside of the second component 6 is typically provided. The second component 6 may alternatively be provided with a hook and loop type fastening means rather than an adhesive.

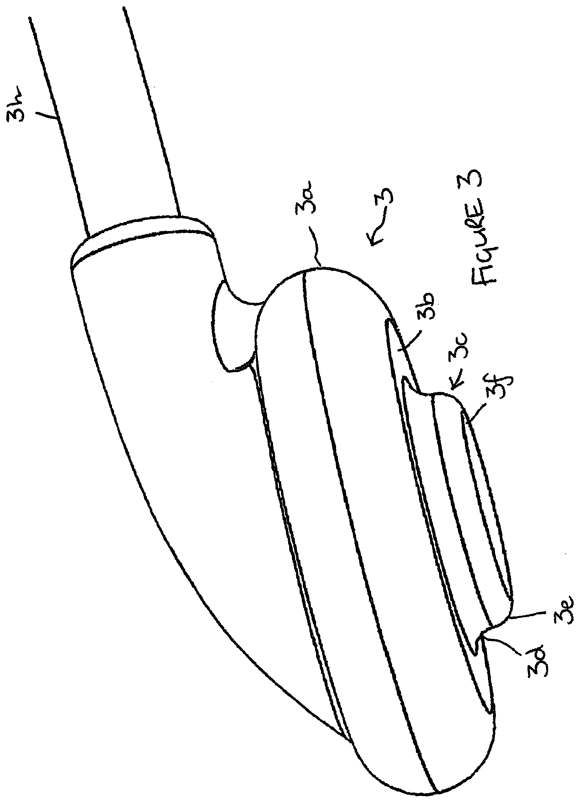

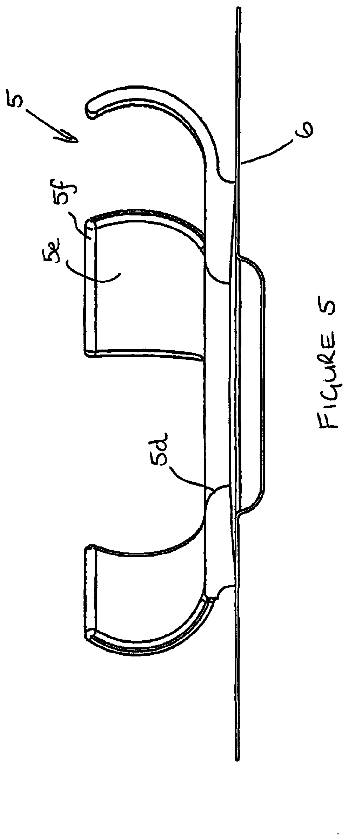

Referring now to Figures 3 to 5: the sensor housing 3 includes an outer portion 3a presenting a curved wall, which in use is gripped by the clips 5e of the attachment member 4, an intermediate portion 3b and a central portion 3c. The intermediate portion 3b presents a substantially planar surface and in use sits adjacent and in abutment with the upper surface of the base 5b of the first component 5. The central portion 3c extends in a direction perpendicular to the intermediate portion 3b with the perimeter of the central portion 3c being bounded by a first wall 3d which is concave and in use sits adjacent and in abutment with the convex wall 5d of the first component and a second wall 3e which is convex. The central portion includes a window 3f which lies parallel to the intermediate portion 3b. The optical element 7 housed in the sensor housing 3 sits behind and spaced from the plane of the window 3f. This is because the optical element 7 need to be spaced apart from the skin by a distance for the detection of infra-red light back scattered by the vasculature of the tissue about the position of attachment of the sensor housing may be detected. The distance is preferably close to 5mm. The range can be from a few mm's to as much as 2.5 cm in muscle type measurements. Whilst the description of the invention is principally in relation to sensors attached to finger and toe pads, the sensor of the invention could be attached to a larger part of the body, such as the calf muscle or forearm. When so used the source to detector spacing is typically between 1.5 and 5 cm.

It is desirable that the sensor housing 3 should exert a force on the skin of the patient undergoing PPG testing, the force in a direction substantially perpendicular to the skin surface. This is achieved in part by the central portion 3c, through which the near infra-red light is emitted, reflected, and back-scattered, extending beyond the intermediate portion of the sensor housing 3. As mentioned above, the membrane 6 is attached to the underside of the first component 5 of the attachment member 4. The membrane 6 also extends over and preferably attaches to the surface 3f of the sensor housing 3.

When the assembled device 1 is attached to the digit 2 of a patient as shown in Figure 1, the surface 3f of the sensor housing 3 is caused to engage and press on the surface of skin of the digit 2. The degree of tension applied to the tabs 6b of the flexible membrane largely determines the pressure exerted on the skin beneath the surface 3f. Preferably, the force exerted by the sensor on the skin is approximately 0.4N over a surface area of circle having a diameter of approximately 1cm.

The attachment member 4 holds the sensor housing 3 in position on a radial axis extending from the body part to which device 1 is attached. However, the clam shell arrangement of the first component 5 and in particular the clips 5e thereof allow the sensor housing 3 to rotate about the said radial axis. In this way the twisting forces resulting from the weight of the connecting cable 3h are not transmitted to the attachment member 4. In addition, the ability of the sensor housing 3 to rotate is particularly useful in bilateral PPG where the application of the sensor housings should be symmetrical.

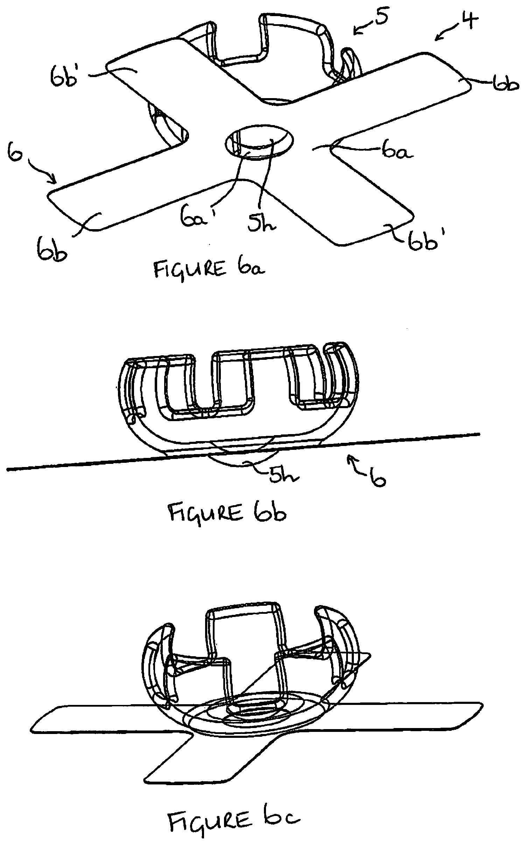

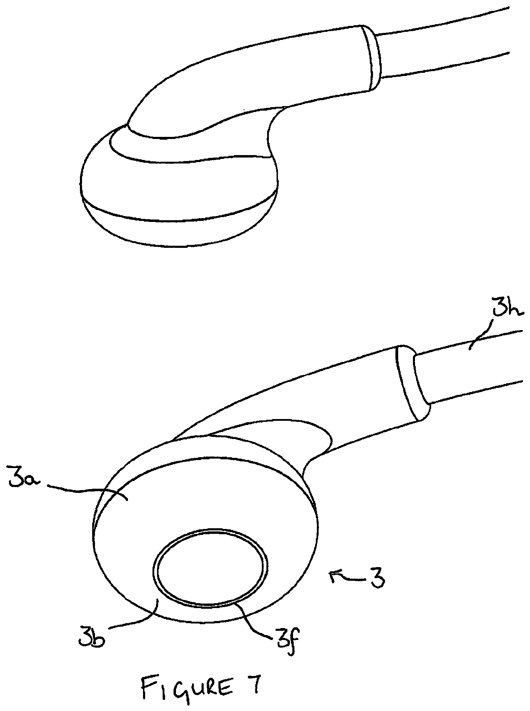

Figures 6a to 6c and Figure 7 illustrate an alternative form of the disposable element 4 and sensor situated in a sensor housing 3. The device differs from that illustrated in Figures 1 to 5 in that instead of the sensor housing 3 including a central portion 3c which, in use, presses onto the skin of the patient, the projecting part is formed within the disposable element 4. The first component 5 of the disposable element 5 is similar to that shown in Figures 1 to 5 and like reference numerals are used to identify like parts. The difference lies in the provision of a protrusion 5h extending from the base 5b of the first component 5. The central part 6a of the second component 6 has a removed portion 6a' with the protrusion 5h extending through the removed portion 6a'. The protrusion 5h shown in Figures 6a to 6c is hemispherical in shape. However, other shapes may be used. For example, the protrusion 5h may have a similar shape to the part 3c showing Figure 3. The component 5 of the disposable part thereby forms a complete physical barrier between the sensor housing 3 and the skin, there being no aperture as seen in 5b.

The particular advantage of the embodiment illustrated in Figures 6a to 7 is to eliminate the requirement of a barrier membrane between protrusion 3f and the skin.

In the sensor housing 3 illustrated in Figure 7 the window 3f lies in the same plane as the intermediate portion 3b. Both the window 3f and the material from which the protrusion 5h are formed are selected such that they are transparent to the wavelength of radiation emitted and detected by an emitter and detector located in the sensor housing 3.

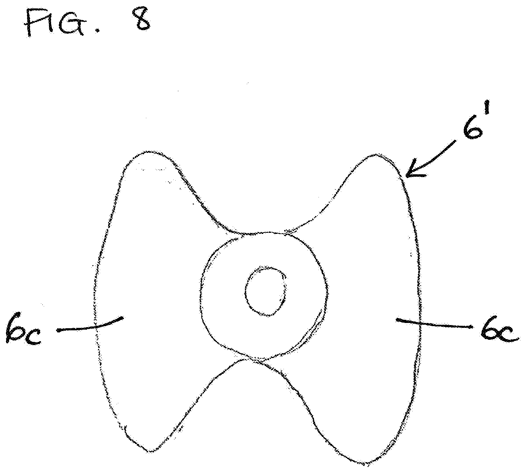

The second component 6 illustrated in Figures 6a to 6c provides for the whole device to be secured to a patient, for example a finger thereof. The tabs 6b, 6b" may have different properties. For example, the tabs 6b may be very flexible so that they may be wrapped around a finger, in the manner illustrated in Figure 1, whereas the tabs 6b' stiff by comparison, thereby tilting of the disposable element 4 and sensor housing 3 attached thereto. As illustrated in Figure 8, the flexible membrane 6' may comprise only two tabs 6c, and these may have any suitable shape, such as the butterfly shape illustrated in Figure 8. The tabs may also be provided with hook and loop type fasteners for attachment of the device to a patient.

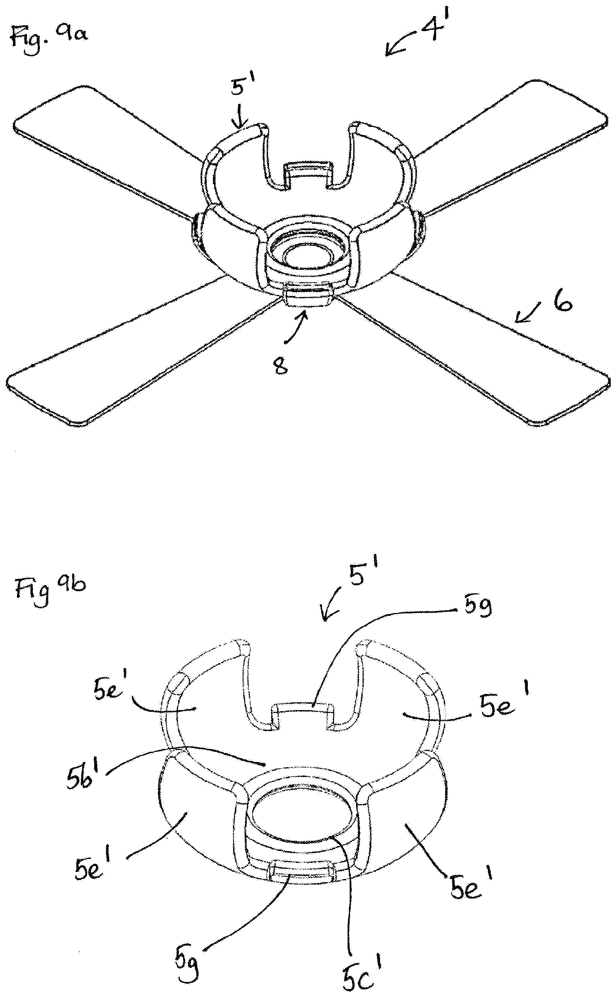

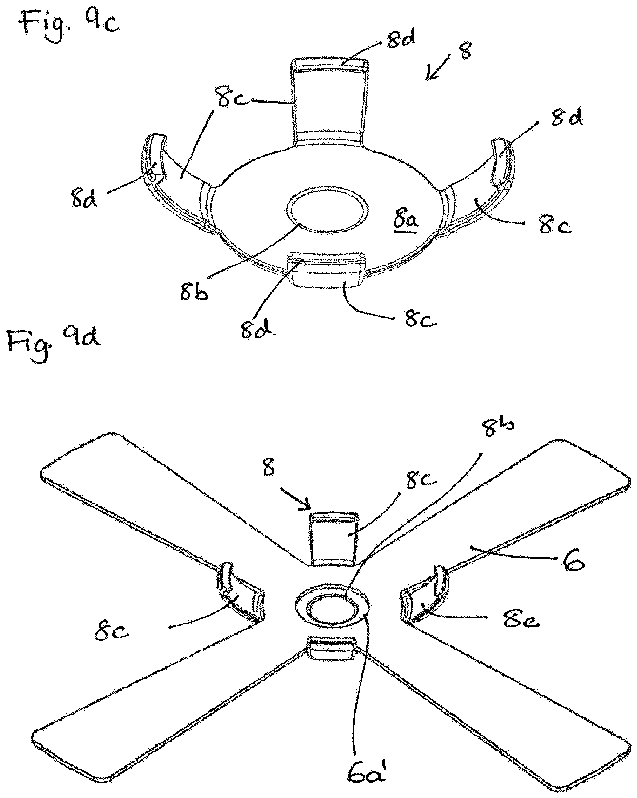

Figures 9a to 9d illustrate an alternative embodiment in which the attachment member 4' comprises an additional third component 8. The first component 5' is a push fit into the third component 8, with the second component, or flexible membrane 6, sandwiched there-between. In this example the first component 5' and third component 8 are held together via frictional forces.

As shown more clearly in Figure 9c, the third component 8 has a base 8a which has a central aperture 8b therein. To the outside of the base 8a is a plurality of clips 8c, four in the illustrated example. The clips are so shaped and dimensioned as to grip the walls of the first component 5'. In the illustrated example, the third component 8 is a one piece plastic moulding, the clips 8c being sufficiently resilient such that when the first component 5 is pressed against the ends 8d of the clips 8c, the clips 8c bend outwards to allow the first component 5' to ride over the ends 8d and then return substantially to the position shown in Figure 9a, exerting a gripping force on the first component 5'. As shown in Figure 9b, the first component 5' of this example differs slightly from the previous example, in that it includes four larger clips 5e' for gripping the sensor housing 3, and two smaller clips 5g which facilitate engagement of the clips 8c of the third component 8. As illustrated in Figure 9a the clips 8c grip the first component 5' in the spaces in-between the clips 5e'. In this example, where the flexible membrane includes an adhesive for attachment to the skin of a patient, and the adhesive layer is protected by a backing layer, the backing layer is preferably perforated adjacent the central aperture 6a' to allow removal of the backing layer once the flexible membrane has been sandwiched between the first component 5' and the third component 8. Both the flexible membrane 6 and the backing layer have an aperture 6a' at the centre to allow passage of the diagnostic radiation from the sensor housing 3 to the patient without further absorption.

## Claims

1. A sensing device (1) comprising a sensor housing (3) mounting sensor elements and a attachment member (4), the attachment member (4) comprising first (5) and second (6) components, the first component (5) configured to receive the sensor housing (3) therein and to permit rotational movement between the attachment member (4) and the sensor housing (3) and to restrict relative movement between the attachment member (4) and the sensor housing (3) in the axial direction of the sensor housing (3), and the second component comprising a flexible membrane (6) that is attachable to an underside of the first component (5), the flexible membrane (6) extending at least in part beyond an outer edge of the first component (5), the flexible membrane (6) comprising attachment means for attachment of the attachment member (4) to a patient, and wherein the attachment member (4) is adapted to allow passage of radiation from the sensor housing (3) to the patient, and wherein the flexible membrane (6) comprises an upper side and an underside, and wherein, in use, the underside of the flexible membrane (6) is in contact with the patient's skin, and includes means for attachment to the patient's skin, and the upper side of the flexible membrane engages with the underside of the first component of the attachment member, and the sensor housing exerts a force on the patient's skin.

2. A sensing device according to Claim 1, wherein the attachment means comprises at least one of the group consisting of: a hook and loop type fastener; and an adhesive surface at least to the side of the flexible membrane (6) that is distal from the first component (5).

3. A sensing device according to Claim 2, wherein the adhesive of the adhesive surface to the side of the flexible membrane (6) that is distal from the first component (5) has an adhesion within a certain range, and adhesion is defined by a peel adhesion strength to steel selected from the group consisting of: 0.1kg/25mm width to 1kg/25mm width; and 0.125kg to 0.6kg/25mm width.

4. A sensing device according to any of Claims 1 to 3, wherein the part of the flexible membrane (6) that extends beyond the outer edge of the first component (5) comprises a plurality of tabs (6b).

5. A sensing device according to any preceding claim, wherein the attachment member (4) comprises a third component (8) in the form of clip means, wherein the third component (8) is shaped and dimensioned to receive the first component (5) in a push-fit arrangement, and wherein, in use, the flexible member (6) is located in-between the first component (5) and the third component (8), such that the third component (8) attaches the flexible membrane (6) to the underside of the first component (5).

6. A sensing device according to any of Claims 1 to 4, wherein the flexible membrane (6) is attached to the underside of the first component (5) by means selected from the group consisting of adhesive and welding.

7. A sensing device according to any preceding claim, wherein the flexible membrane (6) comprises two flexible layers, a first layer for attachment to the underside of the first component (5) and a second layer attached to the first layer and for attachment to the skin of a patient.

8. A sensing device according to Claim 7, wherein the adhesion of the surfaces of the first layer is defined by a peel adhesion to low density polyethylene (LDPE) in the range of 1.1 to 1.5 kg/25.4mm.

9. A sensing device according to any of Claims 1 to 8, wherein the flexible membrane provides an unbroken surface.

10. A sensing device according to any of Claims 1 to 9, wherein the flexible membrane at least in the region where radiation is to pass exhibits low absorption of the wavelength(s) of radiation emitted and sensed by components housed in the sensor housing.

11. A sensing device according to any preceding claim, wherein the flexible membrane includes a hole situated substantially centrally therein.

12. A sensing device according to any preceding claim, wherein the first component comprises a plurality of clips which are resilient and which are so shaped and dimensioned as to grip the sensor housing when the sensor housing is mounted within the clips, and wherein the clips are sufficiently resilient to allow the sensor housing to ride over free ends of the clips when the sensor housing is pressed into or out of the first component of the attachment member.

13. A sensing device according to any preceding claim, wherein the first component of the attachment member includes a protruding part extending from the base of said attachment member, the protruding part formed of a material that exhibits low absorption of wavelengths of radiation emitted and sensed by components housed in the sensor housing.

14. A sensing device according to Claim 11 or claim 10 or 12 when dependent on Claim 9, wherein the first component of the attachment member includes a hole configured to receive a protruding part of the sensor housing; or
wherein the first component of the attachment member includes a hole configured to receive a protruding part of the sensor housing and
wherein, when the sensor housing is mounted in the first component of the attachment member, the protruding part of the sensor housing extends through the hole in the first component; or
wherein the first component of the attachment member includes a hole configured to receive a protruding part of the sensor housing and
wherein the protruding part of the sensor housing extends beyond the surface of the first component that is distal from the sensor housing.

15. A sensing device according to Claim 14, wherein the flexible membrane is attached to the underside of the first component around the hole and the flexible membrane covers the hole.

## Patentansprüche

1. Erfassungsvorrichtung (1), umfassend ein Sensorgehäuse (3), das Sensorelemente montiert, und ein Anbringungsbauteil (4), wobei das Anbringungsbauteil (4) eine erste (5) und eine zweite (6) Komponente umfasst, wobei die erste Komponente (5) dazu konfiguriert ist, das Sensorgehäuse (3) darin aufzunehmen und eine Drehbewegung zwischen dem Anbringungsbauteil (4) und dem Sensorgehäuse (3) zuzulassen und eine relative Bewegung zwischen dem Anbringungsbauteil (4) und dem Sensorgehäuse (3) in der Achsrichtung des Sensorgehäuses (3) einzuschränken, und die zweite Komponente eine flexible Membran (6) umfasst, die an einer Unterseite der ersten Komponente (5) anbringbar ist, wobei sich die flexible Membran (6) zumindest zum Teil über eine Außenkante der ersten Komponente (5) hinaus erstreckt, wobei die flexible Membran (6) Anbringungsmittel zum Anbringen des Anbringungsbauteils (4) an einem Patienten umfasst, und wobei das Anbringungsbauteil (4) dazu eingerichtet ist, ein Leiten von Strahlung von dem Sensorgehäuse (3) zu dem Patienten zu ermöglichen, und wobei die flexible Membran (6) eine Oberseite und eine Unterseite umfasst, und wobei die Unterseite der flexiblen Membran (6) im Gebrauch in Kontakt mit der Haut des Patienten steht und ein Mittel zum Anbringen an der Haut des Patienten einschließt und die Oberseite der flexiblen Membran die Unterseite der ersten Komponente des Anbringungsbauteils in Eingriff nimmt und das Sensorgehäuse eine Kraft auf die Haut des Patienten ausübt.

2. Erfassungsvorrichtung nach Anspruch 1, wobei das Anbringungsmittel mindestens eines bzw. eine aus der Gruppe umfasst, bestehend aus: einem Befestigungselement vom Klettverschluss-Typ und einer Klebefläche mindestens zu der Seite der flexiblen Membran (6), die von der ersten Komponente (5) distal ist.

3. Erfassungsvorrichtung nach Anspruch 2, wobei der Klebstoff der Klebefläche zu der Seite der flexiblen Membran (6), die distal von der ersten Komponente (5) ist, eine Adhäsion innerhalb eines bestimmten Bereichs aufweist und die Adhäsion durch eine Schälfestigkeit an Stahl definiert ist, die ausgewählt ist aus der Gruppe bestehend aus: 0,1 kg/25-mm-Breite bis 1 kg/25-mm-Breite und 0,125 kg bis 0,6 kg/ 25-mm-Breite.

4. Erfassungsvorrichtung nach einem der Ansprüche 1 bis 3, wobei der Teil der flexiblen Membran (6), der sich über die Außenkante der ersten Komponente (5) hinaus erstreckt, eine Vielzahl von Laschen (6b) umfasst.

5. Erfassungsvorrichtung nach einem vorhergehenden Anspruch, wobei das Anbringungsbauteil (4) eine dritte Komponente (8) in der Form eines Klammermittels umfasst, wobei die dritte Komponente (8) dazu geformt und bemessen ist, die erste Komponente (5) in einer Einpressanordnung aufzunehmen, und wobei sich das flexible Bauteil (6) im Gebrauch zwischen der ersten Komponente (5) und der dritten Komponente (8) befindet, so dass sich die dritte Komponente (8) an der flexiblen Membran (6) an der Unterseite der ersten Komponente (5) anschließt.

6. Erfassungsvorrichtung nach einem der Ansprüche 1 bis 4, wobei die flexible Membran (6) an der Unterseite der ersten Komponente (5) durch Mittel angebracht wird, die aus der Gruppe bestehend aus Klebstoff und Schweißen ausgewählt sind.

7. Erfassungsvorrichtung nach einem vorhergehenden Anspruch, wobei die flexible Membran (6) zwei flexible Schichten umfasst, eine erste Schicht zum Anbringen an der Unterseite der ersten Komponente (5) und eine zweite Schicht, die an der ersten Schicht angebracht ist und zum Anbringen an der Haut des Patienten.

8. Erfassungsvorrichtung nach Anspruch 7, wobei die Adhäsion der Flächen der ersten Schicht durch eine Schälfestigkeit an Polyethylen niedriger Dichte (LDPE) im Bereich von 1,1 bis 1,5 kg/25,4 mm definiert ist.

9. Erfassungsvorrichtung nach einem der Ansprüche 1 bis 8, wobei die flexible Membran eine ununterbrochene Fläche bereitstellt.

10. Erfassungsvorrichtung nach einem der Ansprüche 1 bis 9, wobei die flexible Membran zumindest in der Region, in der Strahlung zu leiten ist, eine geringe Absorption der Wellenlänge(n) von Strahlung aufweist, die emittiert und von Komponenten erfasst wird, die in dem Sensorgehäuse untergebracht sind.

11. Erfassungsvorrichtung nach einem vorhergehenden Anspruch, wobei die flexible Membran ein Loch einschließt, das sich im Wesentlichen zentral darin befindet.

12. Erfassungsvorrichtung nach einem vorhergehenden Anspruch, wobei die erste Komponente eine Vielzahl von Klammern umfasst, die elastisch sind und die so geformt und bemessen sind, dass sie das Sensorgehäuse greifen, wenn das Sensorgehäuse innerhalb der Klammern montiert ist, und wobei die Klammern ausreichend elastisch sind, um zu ermöglichen, dass das Sensorgehäuse über freie Enden der Klammern gleitet, wenn das Sensorgehäuse in die oder aus der ersten Komponente des Anbringungsbauteils gedrückt wird.

13. Erfassungsvorrichtung nach einem vorhergehenden Anspruch, wobei die erste Komponente des Anbringungsbauteils einen hervorstehenden Teil einschließt, der sich von der Basis des besagten Anbringungsbauteils erstreckt, wobei der hervorstehende Teil aus einem Material hergestellt ist, das eine geringe Absorption von Wellenlängen von Strahlung aufweist, die emittiert und von Komponenten erfasst wird, die in dem Sensorgehäuse untergebracht sind.

14. Erfassungsvorrichtung nach Anspruch 11 oder Anspruch 10 oder 12 bei Abhängigkeit von Anspruch 9, wobei die erste Komponente des Anbringungsbauteils ein Loch einschließt, das dazu konfiguriert ist, einen hervorstehenden Teil des Sensorgehäuses aufzunehmen; oder
wobei die erste Komponente des Anbringungsbauteils ein Loch einschließt, das dazu konfiguriert ist, einen hervorstehenden Teil des Sensorgehäuses aufzunehmen, und
wobei, wenn das Sensorgehäuse in der ersten Komponente des Anbringungsbauteils montiert ist, sich der hervorstehende Teil des Sensorgehäuses durch das Loch in der ersten Komponente erstreckt; oder
wobei die erste Komponente des Anbringungsbauteils ein Loch einschließt, das dazu konfiguriert ist, einen hervorstehenden Teil des Sensorgehäuses aufzunehmen, und
wobei sich der hervorstehende Teil des Sensorgehäuses über die Fläche der ersten Komponente hinaus erstreckt, die distal von dem Sensorgehäuse ist.

15. Erfassungsvorrichtung nach Anspruch 14, wobei die flexible Membran an der Unterseite der ersten Komponente um das Loch angebracht wird und die flexible Membran das Loch abdeckt.

## Revendications

1. Un dispositif de détection (1) comprenant un boîtier de capteur (3) pour monter des éléments de capteur et un élément de fixation (4), l'élément de fixation (4) comprenant un premier (5) et un deuxième (6) composants, le premier composant (5) configuré pour recevoir le boîtier de capteur (3) à l'intérieur de celui-ci et pour permettre un mouvement de rotation entre l'élément de fixation (4) et le boîtier de capteur (3) et pour limiter un mouvement relatif entre l'élément de fixation (4) et le boîtier de capteur (3) dans la direction axiale du boîtier de capteur (3), et le deuxième composant comprenant une membrane flexible (6) qui peut être fixée à une face inférieure du premier composant (5), la membrane flexible (6) qui peut être fixée à une face inférieure du premier composant (5), la membrane flexible (6) comprenant un moyen de fixation pour la fixation de l'élément de fixation (4) à un patient, et dans lequel l'élément de fixation (4) est adapté pour permettre le passage d'un rayonnement depuis le boîtier de capteur (3) vers le patient, et dans lequel la membrane flexible (6) comprend une face supérieure et une face inférieure, et dans lequel, lors d'une utilisation, la face inférieure de la membrane flexible (6) est en contact avec la peau du patient, et inclut un moyen pour la fixation à la peau du patient, et la face supérieure de la membrane flexible s'engage avec la face inférieure du premier composant de l'élément de fixation, et le boîtier de capteur exerce une force sur la peau du patient.

2. Un dispositif de détection selon la revendication 1, dans lequel le moyen de fixation comprend au moins une fixation du groupe constitué de : une fermeture de type à crochets et boucles ; et une surface adhésive au moins sur la face de la membrane flexible (6) qui est distale du premier composant (5).

3. Un dispositif de détection selon la revendication 2, dans lequel l'adhésif de la surface adhésive à la face de la membrane flexible (6) qui est distale du premier composant (5) a une adhérence dans une certaine plage, et l'adhérence est définie par une force d'adhérence au pelage sur l'acier sélectionnée dans le groupe constitué de : 0,1 kg/25 mm de large à 1 kg/25 mm de large ; et 0,125 kg à 0,6 kg/ 25 mm de large.

4. Un dispositif de détection selon l'une quelconque des revendications 1 à 3, dans lequel la partie de la membrane flexible (6) qui s'étend au-delà du bord extérieur du premier composant (5) comprend une pluralité de languettes (6b).

5. Un dispositif de détection selon l'une quelconque des revendications précédentes, dans lequel l'élément de fixation (4) comprend un troisième composant (8) ayant la forme d'un moyen de pince, dans lequel le troisième composant (8) a une forme et des dimensions pour recevoir le premier composant (5) dans un agencement à emboîture, et dans lequel, lors d'une utilisation, l'élément flexible (6) est situé entre le premier composant (5) et le troisième composant (8), de telle sorte que le troisième composant (8) fixe la membrane flexible (6) à la face inférieure du premier composant (5).

6. Un dispositif de détection selon l'une quelconque des revendications 1 à 4, dans lequel la membrane flexible (6) est fixée à la face inférieure du premier composant (5) par un moyen sélectionné dans le groupe constitué d'adhésif et de soudure.

7. Un dispositif de détection selon l'une quelconque des revendications précédentes, dans lequel la membrane flexible (6) comprend deux couches flexibles, une première couche pour la fixation à la face inférieure du premier composant (5) et une deuxième couche fixée à la première couche et pour la fixation à la peau d'un patient.

8. Un dispositif de détection selon la revendication 7, dans lequel l'adhérence des surfaces de la première couche est définie par une adhérence au pelage sur un polyéthylène faible densité (LDPE) dans la plage de 1,1 à 1,5 kg/25,4 mm.

9. Un dispositif de détection selon l'une quelconque des revendications 1 à 8, dans lequel la membrane flexible fournit une surface ininterrompue.

10. Un dispositif de détection selon l'une quelconque des revendications 1 à 9, dans lequel la membrane flexible, au moins dans la région où le rayonnement va passer, présente la ou les longueur(s) d'onde de rayonnement émise(s) et détectée(s) par des composants logés dans le boîtier de capteur.

11. Un dispositif de détection selon l'une quelconque des revendications précédentes, dans lequel la membrane flexible inclut un trou situé sensiblement centralement à l'intérieur de celle-ci.

12. Un dispositif de détection selon l'une quelconque des revendications précédentes, dans lequel le premier composant comprend une pluralité de pinces qui sont élastiques et ont une forme et des dimensions pour saisir le boîtier de capteur quand le boîtier de capteur est monté à l'intérieur des pinces, et dans lequel les pinces sont suffisamment élastiques pour permettre au boîtier de capteur de chevaucher des extrémités libres des pinces quand le boîtier de capteur est pressé pour entrer ou sortir du premier composant de l'élément de fixation.

13. Un dispositif de détection selon l'une quelconque des revendications précédentes, dans lequel le premier composant de l'élément de fixation inclut une partie saillante s'étendant de la base dudit élément de fixation, la partie saillante formée d'un matériau qui présente une faible absorption de longueurs d'onde de rayonnement émises et détectées par des composants logés dans le boîtier de capteur.

14. Un dispositif de détection selon la revendication 11 ou la revendication 10 ou 12 quand elle dépend de la revendication 9, dans lequel le premier composant de l'élément de fixation inclut un trou configuré pour recevoir une partie saillante du boîtier de capteur ; ou
dans lequel le premier composant de l'élément de fixation inclut un trou configuré pour recevoir une partie saillante du boîtier de capteur et
dans lequel, quand le boîtier de capteur est monté dans le premier composant de l'élément de fixation, la partie saillante du boîtier de capteur s'étend à travers le trou dans le premier composant ; ou
dans lequel le premier composant de l'élément de fixation inclut un trou configuré pour recevoir une partie saillante du boîtier de capteur et
dans lequel la partie saillante du boîtier de capteur s'étend au-delà de la surface du premier composant qui est distale du boîtier de capteur.

15. Un dispositif de détection selon la revendication 14, dans lequel la membrane flexible est fixée à la face inférieure du premier composant autour du trou et la membrane flexible couvre le trou.
